# EUROPEAN PATENT SPECIFICATION

(11) **EP 3 073 843 B1**
(45) Date of publication and mention of the grant of the patent: **11.10.2017**
(21) Application number: 14805493.5
(22) Date of filing: 28.11.2014
(51) Int. Cl.: A23L 33/15, A23L 33/16, A61K 31/14, A61K 31/355, A61K 31/375, A61K 31/4415, A61K 33/30

(54) **COMPOSITION TO ALLEVIATE DETRIMENTAL EFFECTS OF ALCOHOL**
ZUSAMMENSETZUNG ZUR LINDERUNG DER SCHADENSWIRKUNG VON ALKOHOL
COMPOSITION POUR ATTÉNUER LES EFFETS NUISIBLES DE L'ALCOOL

(30) Priority: 29.11.2013 EP 13005560
(43) Date of publication of application: 05.10.2016
(73) Proprietor: Merz Pharma GmbH & Co. KGaA, 60318 Frankfurt (DE)
(72) Inventor: ARESLAN, Nabila, 60318 Frankfurt am Main (DE); FUELBIER, Sandra, 63075 Offenbach (DE); HAUPTMEIER, Bernhard, 63571 Gelnhausen (DE); HECKMANN, Jutta, 60431 Frankfurt am Main (DE)
(74) Representative: Wallinger, Michael
(86) International application number: PCT/EP2014/003196
(87) International publication number: WO 2015/078593

(56) References cited:
- WO-A2-2009/118726
- WO-A2-2010/118405
- CN-A- 103 190 557
- US-A1- 2001 033 881
- US-A1- 2001 043 956
- US-A1- 2002 119 181
- US-A1- 2003 211 172
- US-A1- 2011 150 968

## Description

### FIELD OF THE INVENTION

The present invention relates to a composition that is suitable to alleviate the toxic effects of alcohol, in particular the toxic effects to the liver such that liver function is protected and maintained. Said composition may be used as or in a dietary supplement or in form of a pharmaceutical composition.

### BACKGROUND OF THE INVENTION

Ethyl alcohol or ethanol is often found in alcoholic beverages intended for ingestion such as beer, wine, and spirits. Consumption of ethyl alcohol may be followed by a condition referred to as a hangover. Common hangover symptoms include headache, dehydration, nausea, lethargy, congestion, diarrhea, and/or fever. These symptoms may be particularly severe, especially after heavy consumption of alcoholic beverages. While typically not life-threatening, these symptoms are unpleasant and may interfere with a person's performance at a job or at home. The hangover is due mainly to the toxic effects of acetaldehyde, a byproduct of the breakdown of ethyl alcohol in the liver. The hangover symptoms are unpleasant, and seem to vary in intensity according to the individual and the amount of alcoholic beverages consumed.

Alcohol consumption has additionally well-known detrimental effects on nutrition. The damage is done by the toxic effects of alcohol and by the nutrient deficiencies caused by alcohol in the body. Liver damage is the best known result of longterm alcohol abuse. The liver will swell with acute intoxication, sometimes painfully, and will show fatty infiltration and enlargement if alcoholic beverage ingestion continues regularly.

It is known that vitamins A, D, E, K, B1 and folic acid may be used in dietary supplements against liver damages.

It is further known to use plant extract such as silymarin (carduus marianus; milk thistle), or amino acids such as ornithine aspartate or other amino compounds such as choline to support liver function.

A variety of dietary supplements is known that contain one or more of the above compounds in order to support liver function or to alleviate symptoms associated with alcohol consumption.

For example, WO 2012/120036 A1 relates to an orthomolecular agent for countering the consequences of alcohol consumption. The agent comprises a cysteine compound, a magnesium salt, a zinc salt, vitamin B1, vitamin B2, vitamin B3, vitamin B5, vitamin B6 and vitamin B12.

US 2007/0203315 A1 relates to a dietary nutritional supplement that is designed specifically to address the needs of moderate to heavy alcohol product consumers. The nutritional supplement contains effective amounts of essential components vitamin B2, vitamin B6, vitamin B12 (cobalamin), vitamin B3 (niacin), vitamin B1 (thiamin), calcium, magnesium, vitamin A, vitamin C (ascorbic acid), folic acid, L-cysteine, L-methionine, milk thistle, selenium, dandelion, and molybdenum.

CN 103 190 557 relates to a nutritional product having an effect of repairing liver injuries, which selects roaster liver peptide powder as a primary raw material, with the addition of choline, inositol, zinc gluconate, methionine acid, vitamin B family, vitamin C, vitamin E, mannitol, low-substituted hydroxypropyl cellulose, and talc.

### OBJECTS OF THE INVENTION

In view of the widespread alcohol consumption and the consequences associated therewith, there is a need for providing further compositions in order to ameliorate or alleviate detrimental effects of alcohol consumption. In particular, there is a need for protecting and maintaining liver function, in particular to preventively protect and maintain liver function.

### SUMMARY OF THE INVENTION

According to a ***first aspect*** of the invention, the above stated object may be achieved with a composition comprising or consisting of at least components (i) to (iii):
(i) choline,
(ii) one or more vitamins,
(iii) zinc.

The inventors of the present invention have discovered that a composition comprising or consisting of components (i) to (iii) is suitable to support the well-being of a human, in particular to reduce the detrimental effects of alcohol in the body of a human.

More specifically, the inventors of the present invention have discovered that a composition comprising or consisting of components (i) to (iii) may be suitable to reduce the detrimental effects of alcohol such as hangover and nutritional deficiencies caused by alcohol in the body of a human.

Still more specifically, the inventors of the present invention have discovered that the composition according to the invention allows reducing the detrimental effects of alcohol on liver function, respectively allows protecting and maintaining same, preferably allows to preventively protecting and maintaining liver function.

Consequently, the composition according to the invention may be used in or as a food or dietary supplement or as a pharmaceutical composition.

The beneficial effects have been achieved by the selection of the components (i) to (iii) of the composition according to the invention. Said beneficial effects may be further improved by the selection of appropriate amounts contained therein.

Accordingly, in one embodiment, the invention relates to a composition comprising or consisting of at least components (i) to (iii):
(i) choline in an amount of from 70 - 99 % by weight,
(ii) one or more vitamins in an amount of from 0.5 - 25 % by weight,
(iii) zinc in an amount of from 0.1 - 10 % by weight,
wherein the total amount of choline, the one or more vitamins and zinc amounts to 100 % by weight.

In a more specific embodiment, the invention relates to a ccomposition comprising or consisting of at least components (i) to (iii):
(i) choline in an amount of from 80 - 99 % by weight,
(ii) one or more vitamins in an amount of from 0.5 - 20 % by weight,
(iii) zinc in an amount of from 0.1 - 3 % by weight,
wherein the total amount of choline, the one or more vitamins and zinc amounts to 100 % by weight.

In a further more specific embodiment, the invention relates to a ccomposition comprising or consisting of at least components (i) to (iii):
(i) choline in an amount of from 85 - 99 % by weight,
(ii) one or more vitamins in an amount of from 0.5 - 12 % by weight,
(iii) zinc in an amount of from 0.1 - 3 % by weight,
wherein the total amount of choline, the one or more vitamins and zinc amounts to 100 % by weight.

In one embodiment, at least 50 % by weight or at least 60 % by weight or at least 70 % by weight or at least 80 % by weight or at least 90 % by weight or 100 % by weight of the composition consist of components (i) to (iii), based on the total weight of the composition.

In a further specific embodiment, the invention relates to a composition, wherein the one or more vitamins are selected from the group consisting of vitamin C, vitamin E, vitamin B6, vitamin B9, or a combination of two or more thereof.

In one embodiment, the one or more vitamins are a combination of vitamin C, vitamin E, vitamin B6, and vitamin B9.

In one embodiment,
(i) choline amounts from 85 - 95 % by weight,
(ii) the combination of vitamin C, vitamin E, vitamin B6, and vitamin B9 amounts from 4 - 12 % by weight,
zinc amounts from 0.2 - 3 % by weight.

In one embodiment,
(i) choline amounts from 87 - 95 % by weight,
(ii) vitamin C amounts from 4 - 8 % by weight,
   vitamin E amounts from 0.5 - 2 % by weight,
   vitamin B6 amounts from 0.05 - 0.4 % by weight,
   vitamin B9 amounts from 0.01 - 0.1 % by weight,
(iii) zinc amounts from 0.2 to 3 % by weight.

In another embodiment, the one or more vitamins are a combination of vitamin E, vitamin B6, and vitamin B9.

In one embodiment,
(i) choline amounts from 90 - 99 % by weight,
(ii) the combination of vitamin E, vitamin B6, and vitamin B9 amounts from 0.5 - 8 % by weight,
(iii) zinc amounts from 0.2 - 2 % by weight.

In one embodiment,
(i) choline amounts from 92 - 99 % by weight,
(ii) vitamin E amounts from 0.5 - 6 % by weight,
   vitamin B6 amounts from 0.05 - 0.4 % by weight,
   vitamin B9 amounts from 0.01 - 0.1 % by weight,
(iii) zinc amounts from 0.2 - 2 % by weight.

According to a ***second aspect,*** the invention relates to a dietary supplement comprising or consisting of the composition as defined in the ***first aspect.***

In one embodiment, the dietary supplement is in form of a powder.

In one embodiment, the dietary supplement is in the form of powder which is provided in a stick pack.

In one embodiment, the stick pack has at least two chambers, wherein said vitamin C and choline are in different chambers when the composition comprises vitamin C.

In another embodiment, the dietary supplement is in the form of a tablet, a capsule, an effervescent tablet, or a solution or emulsion.

In one embodiment, the invention relates to a composition as defined in the ***first aspect*** or to a dietary supplement as defined in the ***second aspect,*** wherein the presence of
(a) lactose or ingredients based on lactose, or
(b) ingredients which do not fall under the Arabic term "halal", or (c) ingredients which do not fall under the Jewish term "koscher"
is/are excluded.

In one embodiment, the invention relates to a composition as defined in the ***first aspect,*** or to a dietary supplement as defined in the ***second aspect,*** wherein at least 50 % by weight or at least 60 % by weight or at least 70 % by weight or at least 80 % by weight or at least 90 % by weight or 100 by weight of the composition or the dietary supplement consists of components (i) to (iii), based on the total weight of the composition or the dietary supplement, when the composition or the dietary supplement is in a solid form.

In another embodiment, the invention relates to a composition as defined in the ***first aspect,*** or to a dietary supplement as defined in the ***second aspect,*** wherein from 1 to 5 % by weight, or 5 to 10 % by weight, or 10 to 15 % by weight, or 15 to 20 % by weight, or 20 to 25 % by weight, or 25 to 30 % by weight, or 30 to 35 % by weight, or 35 to 40 % by weight, or 40 to 45 % by weight, or 45 to 50 % by weight, or 50 to 70 % by weight of the composition or the dietary supplement consist of components (i) to (iii), based on the total weight of the composition or the dietary supplement, when the composition or the dietary supplement is in a liquid form.

According to a ***third aspect,*** the invention relates to the use of the composition as defined in the ***first aspect,*** or to the use of the dietary composition as defined in the ***second aspect.***

In one embodiment, the invention relates to the use of a dietary supplement as defined in the ***second aspect*** for a "once daily" application.

In another embodiment, the dietary supplement is used for a "twice daily" application.

In one embodiment, the invention relates to the use of a composition as defined in the ***first aspect,*** or to the use of a dietary supplement as defined in the ***second aspect*** for
(a) supporting a consumer's well-being; or
(b) alleviating a consumer's hangover or hangover symptoms; or
(c) protecting a consumer's liver function; or
(d) maintaining a consumer's liver function.

In one embodiment, the consumer is a human.

In one embodiment, the liver of the human has been or is or will be subjected to alcohol.

In another embodiment, the invention relates to a composition as defined in the ***first aspect*** for use in the
(a) prevention of detrimental effects in a consumer's liver caused by alcohol; or
(b) treatment of detrimental effects in a consumer's liver caused by alcohol.

In another embodiment, the invention relates to a pharmaceutical composition, comprising the composition for use.

The terms in quotation marks as used in the following are defined in the meaning of the invention.

### BRIEF DESCRIPTION OF THE FIGURES

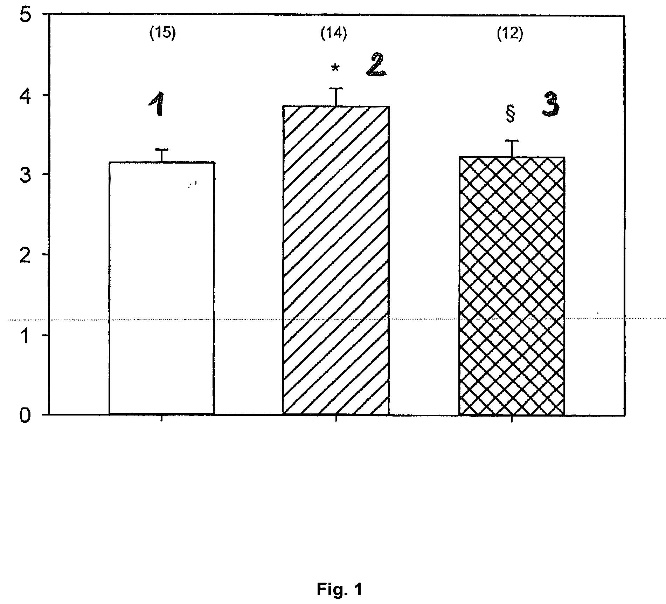

**Figure 1** shows the effect on cholesterol concentration of mice treated with alcohol and a composition according to the invention (group 3) compared to mice treated with alcohol and water (group 2) and water (control; group 1).

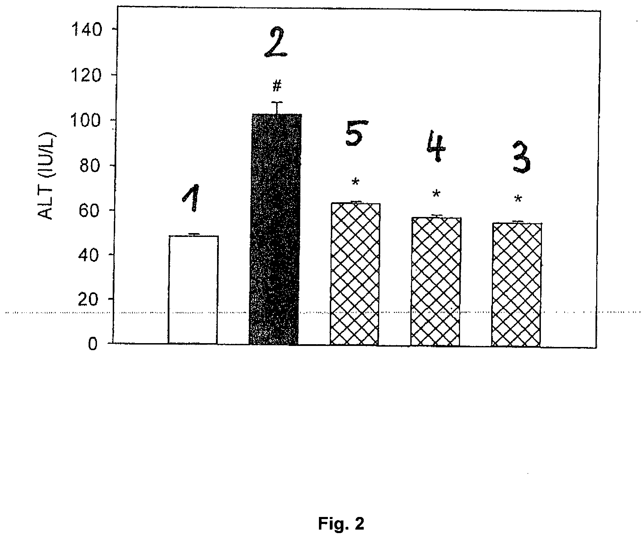

**Figure 2** shows the effect on alanine transferase (ALT) concentration in serum of mice treated with concanavalin A and a composition according to the invention at three different concentrations (groups 3 to 5) compared to mice treated with concanavalin A and water (group 2) and water (group 1; control).

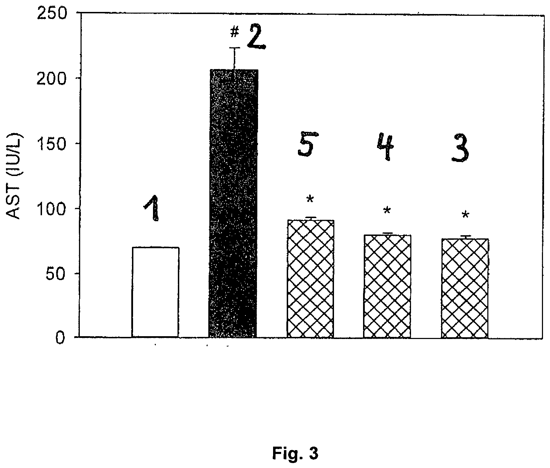

**Figure 3** shows the effect on aspertate transferase (AST) concentration in serum of mice treated with concanavalin A and a composition according to the invention at three different concentrations (groups 3 to 5) compared to mice treated with concanavalin A and water (group 2) and water (group 1; control).

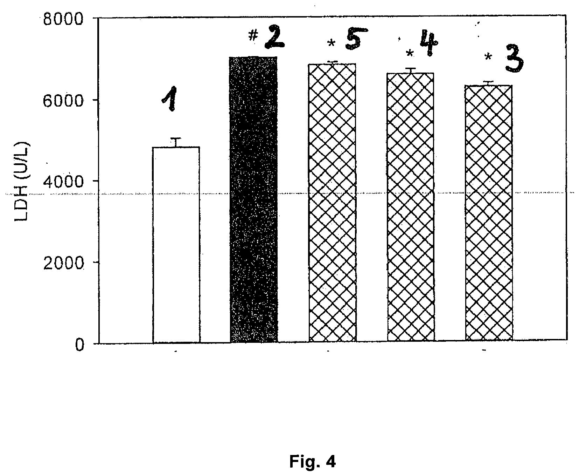

**Figure 4** shows the effect on lactate dehydrogenase (LDH) concentration in serum of mice treated with concanavalin A and a composition according to the invention at three different concentrations (groups 3 to 5) compared to mice treated with concanavalin A and water (group 2) and water (group 1; control).

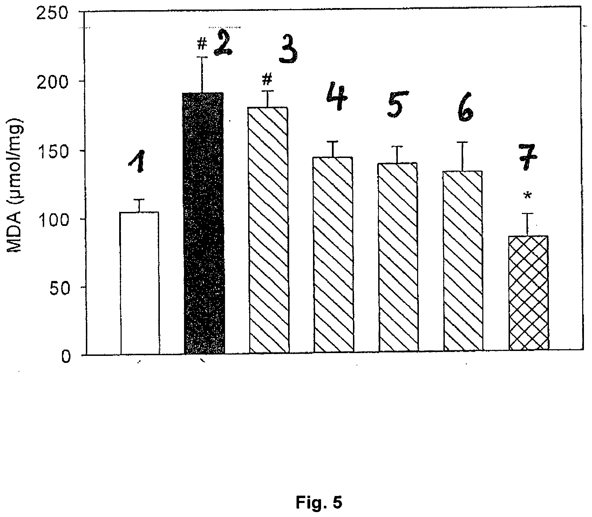

**Figure 5** shows the effect on malondialdehyde concentration in liver tissue of mice treated with alcohol and a composition according to the invention (group 7) compared to mice treated with vitamin B9 (group 6), zinc (group 5), vitamin E (group 4), choline (group 3), water and alcohol (group 2) and pure water (group 1; control).

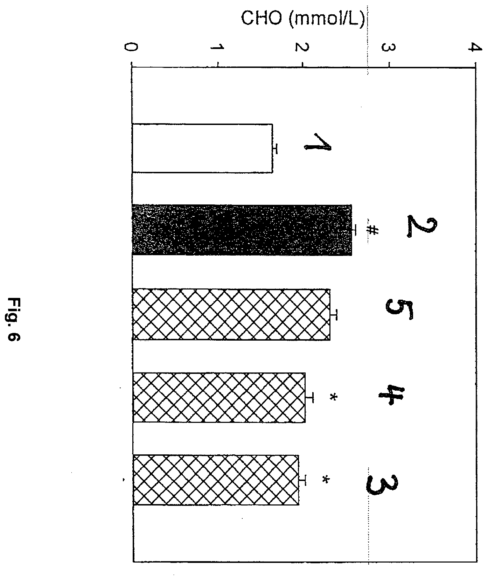

**Figure 6** shows the effect on cholesterol concentration (CHO) in serum of mice treated with alcohol and a composition according to the invention at three different concentrations (groups 3 to 5) compared to mice treated with alcohol and pure water (group 2) and pure water (group 1; control).

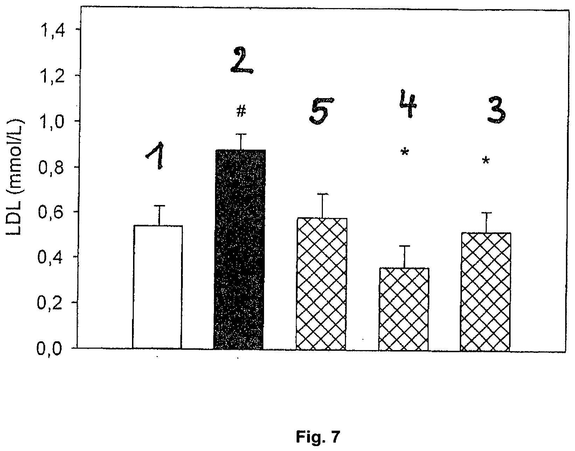

**Figure 7** shows the effect on low density lipoprotein (D-LDL-C) concentration in serum of mice treated with alcohol and a composition according to the invention at three different concentrations (groups 3 to 5) compared to mice treated with alcohol and pure water (group 2) and pure water (group 1; control).

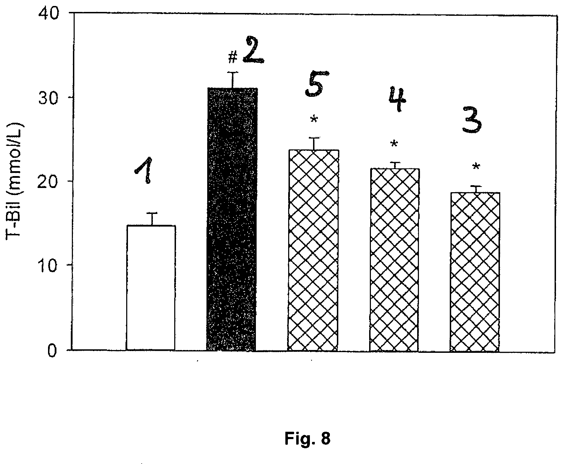

**Figure 8** shows the effect on total bilirubin (T-BiLi) concentration in serum of mice treated with alcohol and a composition according to the invention at three different concentrations (groups 3 to 5) compared to mice treated with alcohol and pure water (group 2) and pure water (group 1; control).

### DETAILED DESCRIPTION OF THE INVENTION

### First aspect: Compositions according to the invention

According to one embodiment according to the ***first aspect,*** the invention relates to a composition comprising or consisting of at least components (i) to (iii):
(i) choline in an amount of from 70 - 99 % by weight,
(ii) one or more vitamins in an amount of from 0.5 - 25 % by weight,
(iii) zinc in an amount of from 0.1 - 10 % by weight,
wherein the total amount of choline, the one or more vitamins and zinc amounts to 100 % by weight.

In one embodiment, said composition comprises or consists of components (i) to (iii):
(i) choline in an amount of from 80 - 99 % by weight,
(ii) one or more vitamins in an amount of from 0.5 - 20 % by weight, e.g. 0.5 to 15 % by weight:
(iii) zinc in an amount of from 0.1 - 3 % by weight,
wherein the total amount of choline, the one or more vitamins and zinc amounts to 100 % by weight.

The term *"choline"* relates to 2-hydroxy-*N,N,N*-trimethylethanaminium (2-hydroxyethyl)trimethylammonium) and a pharmaceutically acceptable counter ion.

The term *"pharmaceutically acceptable*" refers to salts of choline that are physiologically tolerable and do not typically produce untoward reactions when administered to a mammal, e.g., a human. Typically, the term *"pharmaceutically acceptable"* means approved by a regulatory drug and/or food agency of the Federal or a state government or listed in the U.S. Pharmacopeia, other generally recognized pharmacopeia or food regulations like the Food Chemical Codex (FCC) for use in mammals, and more particularly in humans.

Basically, as counter ions the respective pharmaceutically acceptable anions of inorganic or organic acids may be employed.

Examples of pharmaceutically acceptable salts of choline are those formed with the counter ions obtained by deprotonation of hydrochloric, hydrobromic, methanesulfonic, acetic, succinic, maleic, citric acid, and related acids. Further pharmaceutically acceptable salts of choline include, but are not limited to, those formed with the counterions obtained by deprotonation of hydroiodic, perchloric, sulfuric, nitric, phosphoric, carbonic, propionic, glycolic, lactic, gluconic, pyruvic, malonic, fumaric, tartaric, benzoic, carbonic, cinnamic, mandelic, ethanesulfonic, hydroxyethanesulfonic, benezenesulfonic, p-toluene sulfonic, cyclohexanesulfamic, salicyclic, p-aminosalicylic, 2-phenoxybenzoic, aspartic, orotic and 2-acetoxybenzoic acid.

Preferred anions may be selected from halogenides, preferably chloride, or hydroxyl-containing carboxylates, preferably citrate or tartrate, or amino-containing carboxylates, preferably histidinate or aspartate.

The term *"vitamin"* refers to the known vitamins A, B₁, B₂, B₃, B₅, B₆, B₇, B₉, B₁₂, C, D, E, and K.

The term *"zinc"* refers to zinc salts in which the anion of the zinc salt is derived from the anions obtained by deprotonation of acids, preferably the deprotonated acids defined above, i.e. deprotonated hydrochloric, hydrobromic, methanesulfonic, acetic, succinic, maleic, citric acid, and related acids. Further pharmaceutically acceptable salts of zinc include, but are not limited to, those formed with the counterions obtained by deprotonation of hydroiodic, perchloric, sulfuric, nitric, phosphoric, carbonic, propionic, glycolic, lactic, gluconic, pyruvic, malonic, fumaric, tartaric, benzoic, carbonic, cinnamic, mandelic, ethanesulfonic, hydroxyethanesulfonic, benezenesulfonic, p-toluene sulfonic, cyclohexanesulfamic, salicyclic, p-aminosalicylic, 2-phenoxybenzoic, aspartic, orotic and 2-acetoxybenzoic acid.

In a further embodiment of the composition according to the invention, the composition at least comprises or consists of:
(i) choline in an amount of from 85 - 99 % by weight,
(ii) the one or more vitamins in an amount of from 0.5 - 12 % by weight, e.g. 1 - 12 % by weight
(iii) zinc in an amount of from 0.1 - 3 % by weight, e.g. 0.1 - 2 % by weight,
wherein the total amount of choline, the one or more vitamins and zinc amounts to 100 % by weight.

In a further embodiment of the composition according to the invention, the one or more vitamins are selected from the group consisting of vitamin C, vitamin E, vitamin B6, vitamin B9, or a combination of two or more thereof.

In another embodiment of the composition according to the invention, the one or more vitamins are a combination of vitamin C, vitamin E, vitamin B6, and vitamin B9.

The term *"combination"* encompasses a mixture of the vitamins.

In one embodiment, the composition at least comprises or consists of:
(i) choline in an amount of from 85 - 95 % by weight,
(ii) a combination of vitamin C, vitamin E, vitamin B6, and vitamin B9, wherein the combination amounts from 4 - 12 % by weight,
(iii) zinc in an amount of from 0.2 - 3 % by weight, e.g. 0.2 - 2 % by weight,
wherein the total amount of choline, the one or more vitamins and zinc amounts to 100 % by weight.

In a more specific embodiment, the invention relates to a composition comprising or consisting of at least components (i) to (iii):
(i) choline in an amount of from 87 - 95 % by weight, e.g. 85 - 95 % by weight,
(ii) vitamin C in an amount of from 4 - 8 % by weight,
   vitamin E in an amount of from 0.5 - 2 % by weight,
   vitamin B6 in an amount of from 0.05 - 0.4 % by weight,
   vitamin B9 in an amount of from 0.01 - 0.1 % by weight,
(iii) zinc in an amount of from 0.2 - 3 % by weight, e.g. 0.2 - 2 % by weight,
wherein the total amount of choline, the one or more vitamins and zinc amounts to 100 % by weight.

In another embodiment of the composition according to the invention, the one or more vitamins are a combination of vitamin E, vitamin B6, and vitamin B9.

Thus, in one embodiment, the invention relates to a composition comprising or consisting of at least components (i) to (iii):
(i) choline in an amount of from 90 - 99 % by weight,
(ii) a combination of vitamin E, vitamin B6, and vitamin B9, wherein the combination amounts from 0.5 - 8 % by weight, e.g. 1 - 3 % by weight,
(iii) zinc in an amount of from 0.2 - 2 % by weight,
wherein the total amount of choline, the one or more vitamins and zinc amounts to 100 % by weight.

In a more specific embodiment, the invention relates to a composition comprising or consisting of at least components (i) to (iii):
(i) choline in an amount of from 92 - 99 % by weight,
(ii) vitamin E in an amount of from 0.5 - 6 % by weight,
   vitamin B6 in an amount of from 0.05 - 0.4 % by weight,
   vitamin B9 in an amount of from 0.01 - 0.1 % by weight,
(iii) zinc in an amount of from 0.2 - 2 % by weight,
wherein the total amount of choline, the one or more vitamins and zinc amounts to 100 % by weight.

In one embodiment, the composition according to the invention consists of at least components (i) to (iii).

In one embodiment, the composition according to the invention comprises at least components (i) to (iii).

The components (i) to (iii) are commercially available or may be produced according to known methods.

The composition according to the invention may be simply prepared by mixing components (i) to (iii), optionally in presence of further components of the composition according to the invention.

In one embodiment, component (i) is provided in the form of choline chloride, orotate, lactate, acetate or bitartrate.

In another embodiment, zinc according to component (iii) is provided in the form of its sulphate, oxide, carbonate, gluconate, citrate, lactate or acetate, histidinate or aspartate. In one embodiment, zinc is provided in form of its citrate or gluconate.

In another embodiment, component (i) is provided in the form of choline chloride or tartrate, and zinc according to component (iii) is provided in the form of its sulphate, oxide, carbonate, gluconate, citrate, lactate or acetate, histidinate or aspartate.

### Second aspect: Dietary supplement according to the invention

The composition as defined in the ***first aspect*** may be used as or in a dietary supplement.

The term ***"dietary supplement"*** means a compound or a composition that is intended to provide nutrients that may otherwise not be consumed in sufficient quantities by an animate being.

Accordingly, in a ***second aspect,*** the invention relates to dietary supplement comprising or consisting of the composition as defined in the ***first aspect*** according to the invention.

The dietary supplement as defined in the ***second aspect*** or the composition as defined in the ***first aspect*** may be located in one of the following dosage forms: tablet or capsule or granule or powder.

However, it is contemplated that the dietary supplement or the composition according to the invention may be located in other dosage forms such as solution, suspension, elixir, syrup, emulsion, lozenge, pill, sublingual tablet, suppository, oral gels, topical gels, topical creams, topical ointments and topical lotions.

The dietary supplement or the composition according to the invention may be placed in the body of an animate being by, for example, ingesting. It is contemplated that the dietary supplement or the composition according to the invention may enter into the body by other methods.

Accordingly, the dietary supplement or the composition of the present invention may be used by the consumer in a liquid carrier, such as water or a fruit juice (e.g. apple juice or beet juice), or dairy based beverage (e.g. milk or yogurt). The dietary supplement or the composition of the present invention may be formed in a baked good or confection such as a biscuit, cookie, chocolate bar (also known as energy bars). The supplement or the composition of the present invention may alternatively be in tablet form, with one or more tablets constituting a daily dosage. The supplement or the composition may also be in particulate, e.g. powder or granulate, form which may be pre-measured and packaged or packaged in bulk to be measured by the consumer and may added to a carrier liquid, such as water, or may be applied in solid form. Other forms of the supplement or the composition are also possible.

In one embodiment, said dietary supplement is in form of a powder.

The term "powder" encompasses the term *"granules".*

In one embodiment, the powder is provided in a stick pack.

The term *"stick pack"* encompasses a packaging such as a bag or a pouch for the composition or the dietary supplement. For application, the stick pack is opened and the content may be applied directly from said stick pack to the mouth of the consumer, or may be poured into e.g. a fluid such as juice, and may be applied then.

In one embodiment, the stick pack has at least two chambers, each of said chambers accommodating different ingredients of the composition according to the invention.

In one embodiment, when the composition according to the invention comprises vitamin C, said vitamin C and choline are accommodated in different chambers. This is advantageous since during storage a mixture of choline, e.g. choline in the form of the tartrate, and vitamin C may change its physical state, e.g. may become smeary and/or will change its colour which is not acceptable from an aesthetic view.

In another embodiment, the dietary supplement according to the invention is provided in form of a coated or non-coated tablet, a capsule, an effervescent tablet or an orally disintegrating tablet.

The term *"tablet"* defines any solid composition according to the invention comprising the active composition. The term encompasses both compressed formulations and non-compressed formulations. Non compressed formulation can be manufactured e.g. by thermal or melting processes. The tablet may have any shape, which is common in the field of tablets, such as a round shape, a rectangular shape or an oval shape, or a convex shape, or the shape of a disk, or the shape of a bead. The shape may also be irregular. The term also comprises the term *"mini tablet"* and *"micro tablet".* Such term is known from the field of pharmaceutical compositions.

A tablet may be made from powder, granules and / or pellets. The term also encompasses tablet forms such as modified release tablets or immediate release dosage forms. The processing of granules and or pellets into tablets or the formation of immediate and modified release dosage forms is known to a person skilled in the art.

In a further embodiment, the composition or the dietary supplement according to the invention is provided in form of a solution or emulsion. The basis of said solution or emulsion may be water or juice or any liquid that is pharmaceutically acceptable.

In one embodiment, the dietary supplement consists of the composition as defined in the ***first aspect*** of the invention. In another embodiment, the dietary supplement consists of the composition as defined in the ***first aspect*** of the invention, which in turn consists of components (i) to (iii).

The composition as defined in the ***first aspect*** of the invention or the dietary supplement as defined in the ***second aspect*** of the invention may contain further ingredients which commonly are used in dietary supplements.

In one embodiment, the dietary supplement or the composition may further comprise one or more compounds which frequently are used in dietary supplements selected from the group consisting of: vitamin B1, vitamin B2, vitamin B3, vitamin B12, biotin, panthenic acid, folic acid, thiamine, L-cysteine, L-methionine, lutein, riboflavin, proanthocyanidins, ginger root or ginger root extract, gluthathione, bromelain, salicin, milk thistle, selenium, dandelion root powder, molybdenum, sodium, potassium, calcium, magnesium,

In one embodiment, at least 50 % by weight, preferably at least 60 %, more preferred at least 70 %, still more preferred at least 80 % or 90 % of the composition as defined in the ***first aspect*** or the dietary supplement as defined in the ***second aspect*** consist of components (i) to (iii) based on the total weight of the composition or the dietary supplement.

In one embodiment, at least 50 % by weight, preferably at least 60 %, more preferred at least 70 %, still more preferred at least 80 % or 90 % or 100 % of the composition as defined in the ***first aspect*** or the dietary supplement as defined in the ***second aspect*** consist of components (i) to (iii), based on the total weight of the composition or the dietary supplement, when the composition or the dietary supplement is in a solid form.

In one embodiment, from 1 to 5 % by weight, or 5 to 10 % by weight, or 10 to 15 % by weight, or 15 to 20 % by weight, or 20 to 25 % by weight, or 25 to 30 % by weight, or 30 to 35 % by weight, or 35 to 40 % by weight, or 40 to 45 % by weight, or 45 to 50 % by weight, or 50 to 70 % by weight of the composition as defined in the ***first aspect*** or the dietary supplement as defined in the ***second aspect*** consist of components (i) to (iii), based on the total weight of the composition or the dietary supplement, when the composition or the dietary supplement is in a liquid form.

In one embodiment, in the composition as defined in the ***first aspect*** or dietary supplement as defined in the ***second aspect,*** the presence of
(a) lactose or ingredients based on lactose, or
(b) ingredients which do not fall under the Arabic term "halal", or
(c) ingredients which do not fall under the Jewish term "koscher"
is excluded.

The term *"halal"* covers and designates food and drink as permissible according to Islamic law.

The term *"koscher"* covers and designates food and drink as permissible according to Jewish dietary laws.

### Third aspect: Use of the composition as defined in the first aspect or use of the dietary composition as defined in the second aspect

The composition according to the invention as defined in the ***first aspect*** or the dietary supplement as defined in the ***second aspect*** may be used by a consumer for supporting or maintaining or improving its well-being. Since the well-being is defined by the consumer, such well-being is a subjective well-being.

In one embodiment, such *"supporting or maintaining or improving a consumer's well-being"* is independently from alcohol consumption.

In another embodiment, such *"supporting or maintaining or improving a consumer's well-being"* is seen in connection with consumer's alcohol consumption.

The terms *"supporting"* or *"maintaining"* or *"improving"* a consumer's well-being are used interchangeably.

In general, the dietary supplement according to the invention or the composition according to the invention may be consumed by a consumer once or several times per day.

In one embodiment, when the composition or the dietary supplement is provided in powdered form, preferably in a stick pack, said composition or dietary supplement is intended for a "once daily" application.

In one embodiment, when the composition or the dietary supplement is provided in the form of a tablet, or a capsule or an effervescent tablet, said supplement is intended for a "twice daily" application.

In a more specific embodiment, the invention relates to the use of a composition as defined in the ***first aspect*** or a dietary supplement as defined in the ***second aspect*** for ameliorating or alleviating detrimental effects of alcohol consumption in a human.

The terms *"ameliorating"* and *"alleviating"* are used synonymously.

The term *"detrimental effects"* encompasses hangover or hangover symptoms. Common hangover symptoms include headache, dehydration, nausea, lethargy, congestion, diarrhea, and/or fever.

In a further embodiment, the invention relates to the use of a composition as defined in the ***first aspect*** or a dietary supplement as defined in the ***second aspect*** for protecting the liver function of a mammal.

The term *"protecting liver function"* means that the function of the liver is not affected by alcohol.

In a further embodiment, the invention relates to the use of a composition as defined in the ***first aspect*** or a dietary supplement as defined in the ***second aspect*** for maintaining the liver function of a mammal.

The term *"maintaining liver function"* means that the function of the liver is maintained despite alcohol consumption.

The composition as defined in the ***first aspect*** or the dietary supplement as defined in the ***second aspect*** may be used to preventively protect and/or maintain liver function.

In one embodiment, the mammal is a human.

In one embodiment, the liver of said human has been or is or will be subjected to ethyl alcohol.

The composition according to the invention as defined in the ***first aspect*** or the dietary supplement as defined in the ***second aspect*** may be sold over the counter (OTC product).

Moreover, the composition as defined in the ***first aspect*** may be used in the (a) prevention of detrimental effects in a consumer's liver caused by alcohol; or (b) treatment of detrimental effects in a consumer's liver caused by alcohol.

The term *"preventing"* means that the function of the liver is prevented from detrimental effects caused by alcohol.

The term *"treating"* means that a liver that has been affected by alcohol is treated in order to reverse liver damages caused by alcohol such that said damages are reversed or predominantly reversed.

In one embodiment, the composition as defined in the ***first aspect*** invention may be formulated as a pharmaceutical composition.

Thus, in one embodiment, the invention also relates to a pharmaceutical composition comprising the composition as defined in the ***first aspect.***

The pharmaceutical composition may additionally contain besides components (i) to (iii) the ingredients which are mentioned with respect to the dietary supplement as defined in the ***second aspect*** and/or one or more excipients.

The term *"excipient"* encompasses an inactive substance (compound, agent, ingredient) which is used as a carrier for the composition as defined in the ***first aspect.*** The term *"excipient"* also encompasses a pharmaceutically acceptable, physiologically inactive ingredient such as a binder, a filler, a coating-forming compound, a plasticizers for coatings, a compound which masks odors, and the like. Examples of further optional excipients are pigments, flavors, sweeteners, opacifiers, anti-adhesives, preservatives, glidants, lubricants, sorbents. Suitable substances are known in the art. In one embodiment, binders such as sodium methylcellulose or hydroxymethylcellulose may be used. In one embodiment, sweeteners such as sucrose or fructose may be used. In one embodiment, sodium benzoate may be used as a preservative.

In one embodiment, the excipient comprises or consists of microcrystalline cellulose and magnesium stearate.

In another embodiment, if a coated tablet is used, the coating comprises or consists of hypromellose (methylhydroxypropylcellulose), titaniumdioxide, and polyethylenglycol.

In one embodiment, said excipient and/or substances different from components (i) to (iii) is/are present in the composition according to the invention in an amount of less than 50 % by weight, based on the total amount of the composition.

### EXAMPLES

The composition defined in Example 3 made from choline, vitamin E, vitamin B6, vitamin B9, and zinc was used for the following studies.

### Example 1: Liver injury induced by ethanol

Female CD-mice, Crl:CD1 (ICR) (Charles River Deutschland, 97633 Sulzfeld, Germany) weighing 17 - 22 g kept under standard laboratory conditions 5 per cage were used for this study.

The experiment involved three groups (N=15): Group 1: control; Group 2: alcohol (total dose 2.4 g/kg/day); Group 3: alcohol + composition according to the invention.

Treatment involved oral application of water solutions (10 ml/kg) according to the following Table 1:

**Table 1**

| **Group #** | **Composition according to the invention [mg/kg] Days 1 - 30** | **30 % ethanol [ml/kg] Days 17 - 30** | **Pure ethanol [mg/kg] Days 17-30** |
|---|---|---|---|
| 1 | water | -* | - |
| 2 | water | 10 | 2400 |
| 3 | 3500 | 10 | 2400 |

24 hours after the last administration, all animals were sacrificed and their blood was collected for analysis of cholesterol levels using Synchron Cx 5 Beckman.

The results are summarized in **Fig. 1****.** The y-axis shows the level of plasma cholesterol (mMol), the x-axis the type of treatment. Data are shown as mean and SEM as measure of variation.

Mice treated with ethanol 13 days at the dose of 2.4 g/kg/day show a significant increase in plasma levels of cholesterol indicating malfunction of the liver compared to control. In contrast in group treated additionally with the composition according to the invention (17 days before start of administration of alcohol and during alcohol treatment), such increase is not observed. The cholesterol level in this group is in the same magnitude as for control group.

The results were analyzed by ANOVA which revealed a significant effect of treatment followed by Student T test showing significant enhancement of plasma cholesterol after sub-chronic administration of alcohol (* P<0.05) and significant attenuation of this effect by administration of the composition according to the invention ($ P<0.05). The number in brackets indicates number of animals in given group (Fig. **1**).

### Example 2: Liver injury induced by concanavalin A

Female ICR mice weighing 18.0 g-22.0 g reproduced by Beijing (China) Vitalriver Laboratory Animal Technology Co. Ltd. were used for the study.

The composition according to the invention was administered at 3501 mg/kg body weight BW (equivalent to 30 times recommended human dosage), 1167 mg/kg BW (equivalent to 10 times recommended human dosage), 116.7 mg/kg BW (same as the recommended human dosage), negative control group and modeling control group are also set up. The solution was administered orally once a day with an intragastric volume of 0.2 ml/10 g BW for 30 days. On day 30, concanavalin A (ConA) was given i.v. at the dose of 15 mg/kg in a volume of 10 ml/kg BW to produce acute liver injury. After fasting for 8 hours, intraperitoneal injection of 60 mg/kg BW Nembutal solution was used to anaesthetize the mice and blood sampling was performed from abdominal artery. Supernatant serum was taken from the blood sample after it has been centrifuged (at 2500 rpm for 10min), and then used to detect the content of alanine transferase (ALT), aspartate transferase (AST) and lactate dehydrogenase (LDH). Analysis was performed using commercially available kits as follows from Reitman. The results are shown in **Table 2:**

**Table 2 (Experimental groups (N=15 per group)**

| **Group** | **Treatment** |
|---|---|
| 1 | Pure water (*p.o*.), daily for 30 days |
| 2 | Pure water (*p.o*.), daily for 30 days + ConA on day 30 |
| 3 | Composition according to the invention (*p.o*.), 3501 mg/kg, daily for 30 days + ConA on day 30 |
| 4 | Composition according to the invention (*p.o*.), 1167 mg/kg, daily for 30 days + ConA on day 30 |
| 5 | Composition according to the invention (*p.o.*), 116 mg/kg, daily for 30 days + ConA on day 30 |

Results were analyzed using one way ANOVA followed by post-hic pairwise comparison. The composition according to the invention at all used doses completely blocked changes in liver insult as evidenced by effect on serum ALT **(****Fig. 2****),** AST **(****Fig. 3****)** and LDH **(****Fig. 4****).**

**Fig 2****.** shows the effect of a composition according to the invention at 3 doses on increase of ALT concentration in serum produced by Con A. *p<0.01 vs. water + ConA group; #p<0.01 vs. water group. Results are expressed as mean ±SEM.

**Fig 3****.** shows the effect of a composition according to the invention at 3 doses on increase of AST concentration in serum produced by Con A. **p<0.01 vs. water+ConA group; ##p<0.01 vs. water group. Results are expressed as mean ±SEM.

**Fig 4****.** shows the effect of a composition according to the invention at 3 doses on increase of LDH concentration in serum produced by Con A. **p<0.01 vs. water + ConA group; ##p<0.01 vs. water group. Results are expressed as mean ±SEM.

The composition according to the invention prevents in mice chemically-induced liver injury at low doses even equivalent to human dose as calculated per kg. This indicates protective effects of the composition according to the invention on liver function.

### Example 3: Liver injury induced by acute alcohol

Male Kunming (KM) mice (18-22 g of body weight) were used for this study.

The test composition: choline, vitamin E, zinc, vitamin B9 (folic acid) and vitamin B6 (premix of: choline-974 mg, vitamin E-14 mg, zinc-8.9 mg, vitamin B6-2.1 mg and vitamin B9-0.35 mg) were dissolved in saline and were administered by oral gavage for 30 days **(Table 3).** Alcohol was mixed with pure water at 1:1 and administered once orally at the volume of 14 mL/kg (total dose was 7 g/kg) on day 30.

**Table 3 Experimental groups (N=15 per group)**

| **Group** | **Treatment** |
|---|---|
| 1 | Pure water (*p.o*.), daily for 30 days |
| 2 | Pure water (*p.o*.), daily for 30 days + alcohol |
| 3 | Choline (*p.o*.), 668.56 mg/kg, daily for 30 days |
| 4 | Vit E (*p.o*.), 5.96 mg/kg, daily for 30 days |
| 5 | Zinc (*p.o*.), 17.42 mg/kg, daily for 30 days |
| 6 | Vit B9 (Folic acid) (*p.o.*), 0.1 mg/kg, daily for 30 days |
| 7 | Composition according to the invention (*p.o*.), 3501 mg/kg, daily for 30 days |

Sixteen hours after alcohol dose (fasting for 16 hours after alcohol dose), all animals were sacrificed with CO₂ and liver tissue was isolated and weighted, frozen in nitrogen liquid and stored at -80 °C for following detection of malondialdehyde (MDA).

Malondialdehyde (MDA) was co-heated with thiobarbituric acid under acidified condition, producing pink complex. The absorbance peak was at 535 nm. The concentration of MDA was determined using 721 spectrophotometer.

Statistical analysis was performed using the Kruskal-Wallis ANOVA on ranks followed by Dunn's test for pairwise comparison. The difference was considered significant when p< 0.05.

**Fig 5****.** shows the effect of choline, vitamin E, zinc, vitamin B9 (folic acid) and the composition according to the invention on malondialdehyde concentration (MDA; µmol/mg) in liver tissue. **p<0.01 vs. water + ETOH group; ##p<0.01 vs. water group. Results are expressed as mean ±SEM.

Acute treatment with alcohol produced significant increase in malondialdehyde indicating increase lipids peroxidation **(****Fig. 5****).** Treatment with vitamin E, zinc, and vitamin B9 produced a trend for attenuation of this effect as compared to pure water group. However, the only significant effect as compared to alcohol alone group was produced by the composition according to the invention which is also illustrated by complete reversal of alcohol effect. This indicates that ingredients of the composition according to the invention may produce a synergistic effect which is much stronger then ingredients given separately.

### Example 4: Liver injury produced by sub-chronic alcohol administration

Female ICR mice weighing 18.0 g - 22.0 g reproduced by Beijing (China) Vitalriver Laboratory Animal Technology Co. Ltd. were used for the study.

The composition according to the invention or water were administered orally **(Table 4)** on daily basis for 45 days.

On days 30-45, 30% ethanol, 10 ml/kg (ethanol density 0.8 g/ml dose of 2400 mg/kg) was given orally to mice four hours after administration of composition according to the invention. Four hours after the last dose animals were injected intraperitoneally with 60 mg/kg BW Nembutal solution.

**Table 4 Experimental groups (N=15 per group)**

| **Group** | **Treatment** |
|---|---|
| 1 | Pure water (*p.o*.), daily for 30 days |
| 2 | Pure water (*p.o.*), daily for 30 days + alcohol on days 15-30 |
| 3 | Composition according to the invention (*p.o*.), 3501 mg/kg, daily for 30 days + Alcohol on days 15-30 |
| 4 | Composition according to the invention (*p.o*.), 1167 mg/kg, daily for 30 days + Alcohol on days 15-30 |
| 5 | Composition according to the invention (*p.o*.), 116 mg/kg, daily for 30 days + Alcohol on days 15-30 |

Sample of the blood was taken from abdominal artery, sample the supernatant of serum was collected after centrifuging the blood sample (at 2,500 rpm for 10 min), and then used to detect the content of cholesterol (CHO), low density lipoprotein (D-LDL-C) and total bilirubin (T-BiLi) in the serum according to their respective detection kit manual. Results were analyzed using one way ANOVA followed by post-hic pairwise comparison.

Sub-chronic treatment with alcohol produced expected changes i.e. increase in CHO, LDL and T-BiLi. Treatment with the composition according to the invention produced dose dependent reversal of these changes **(****Fig. 6****,** **7****,** **8****).**

**Fig 6****.** shows the effect of composition according to the invention at 3 doses on increase of CHO concentration (mmol/L; y-axis) in serum produced by subchronic alcohol treatment. **p<0.01 vs. water + alcohol group; ##p<0.01 vs. water group. Results are expressed as mean ±SEM.

**Fig 7****.** shows the effect of composition according to the invention at 3 doses on increase of LDL concentration (mmol/L; y-axis) in serum produced by subchronic alcohol treatment. **p<0.05 vs. water + alcohol group; ##p<0.05 vs. water group. Results are expressed as mean ±SEM.

**Fig 8****.** shows the effect of the composition according to the invention at 3 doses on increase of T-Bili concentration (mmol/L; y-axis) in serum produced by sub-chronic alcohol treatment. **p<0.01 vs. water + alcohol group; ##p<0.01 vs. water group. Results are expressed as mean ±SEM.

The results show a clear protective effect of the composition according to the invention against liver injury produced by sub-chronic treatment with alcohol.

## Claims

1. Composition comprising at least components (i) to (iii):
(i) choline in an amount of from 70 - 99 % by weight,
(ii) one or more vitamins in an amount of from 0.5 to 25 % by weight,
(iii) zinc in an amount of from 0.1 to 10 % by weight,
wherein the total amount of choline, the one or more vitamins and zinc amounts to 100 % by weight.

2. Composition according to claim 1, comprising at least components (i) to (iii):
(i) choline in an amount of from 80 - 99 % by weight,
(ii) one or more vitamins in an amount of from 0.5 to 20 % by weight,
(iii) zinc in an amount of from 0.1 - 3 % by weight; or
comprising at least components (i) to (iii):
(i) choline in an amount of from 85 - 99 % by weight,
(ii) one or more vitamins in an amount of from 0.5 to 12 % by weight,
(iii) zinc in an amount of from 0.1 - 3 % by weight.

3. Composition of any one of the preceding claims, wherein the one or more vitamins are selected from the group consisting of vitamin C, vitamin E, vitamin B6, vitamin B9, or a combination of two or more thereof.

4. Composition of claim 3, wherein the one or more vitamins are selected from the group consisting of vitamin C, vitamin E, vitamin B6, vitamin B9, or a combination of two or more thereof, and wherein the one or more vitamins are a combination of vitamin C, vitamin E, vitamin B6, and vitamin B9; or
wherein the one or more vitamins are selected from the group consisting of vitamin C, vitamin E, vitamin B6, vitamin B9, or a combination of two or more thereof, and wherein the one or more vitamins are a combination of vitamin C, vitamin E, vitamin B6, and vitamin B9, and wherein
(i) choline amounts from 85 - 95 % by weight,
(ii) the combination of vitamin C, vitamin E, vitamin B6, and vitamin B9 amounts from 4 - 12 % by weight,
(iii) zinc amounts from 0.2 - 3 % by weight; or
wherein
(i) choline amounts from 87 - 95 % by weight,
(ii) vitamin C amounts from 4 - 8 % by weight,
vitamin E amounts from 0.5 - 2 % by weight,
vitamin B6 amounts from 0.05 - 0.4 % by weight,
vitamin B9 amounts from 0.01 - 0.1 % by weight.

5. Composition of claim 3, wherein the one or more vitamins are a combination of vitamin E, vitamin B6, and vitamin B9; or
wherein the one or more vitamins are a combination of vitamin E, vitamin B6, and vitamin B9, and wherein
(i) choline amounts from 90 - 99 % by weight,
(ii) the combination of vitamin E, vitamin B6, and vitamin B9 amounts from 0.5 - 8 % by weight,
(iii) zinc amounts from 0.2 - 2 % by weight; or
wherein
(i) choline amounts from 92 - 99 % by weight
(ii) vitamin E amounts from 0.5 - 6 % by weight,
vitamin B6 amounts from 0.05 - 0.4 % by weight,
vitamin B9 amounts from 0.01 - 0.1 % by weight,
(iii) zinc amounts from 0.2 - 2 % by weight.

6. Composition according to any one of the preceding claims, wherein at least 50
% by weight or at least 60 % by weight or at least 70 % by weight or at least 80 % by weight or at least 90 % by weight or 100 by weight of the composition consists of components (i) to (iii), based on the total weight of the composition, when the composition is in a solid form; or
wherein from 1 to 5 % by weight, or 5 to 10 % by weight, or 10 to 15 % by weight, or 15 to 20 % by weight, or 20 to 25 % by weight, or 25 to 30 % by weight, or 30 to 35 % by weight, or 35 to 40 % by weight, or 40 to 45 % by weight, or 45 to 50 % by weight, or 50 to 70 % by weight of the composition consist of components (i) to (iii), based on the total weight of the composition, when the composition is in a liquid form.

7. Dietary supplement comprising the composition as defined in any one of the preceding claims.

8. Dietary supplement of claim 7 in form of a powder, or
in form of a powder, wherein the powder is provided in a stick pack.

9. Dietary supplement of claim 8, in form of a powder, wherein the powder is provided in a stick pack, and wherein the stick pack has at least two chambers, wherein said vitamin C and choline are in different chambers when the composition comprises vitamin C.

10. Dietary supplement of claim 7 in form of a tablet, a capsule, an effervescent tablet, or a solution or emulsion.

11. Composition as defined in any one of claims 1 to 6, or dietary supplement as defined in any one of claims 7 to 10, wherein the presence of
(a) lactose or ingredients based on lactose, or
(b) ingredients which do not fall under the Arabic term "halal", or
(c) ingredients which do not fall under the Jewish term "koscher"
is excluded.

12. Use of a dietary supplement as defined in claim 9 for a "once daily" application, or use of a dietary supplement as defined in claim 10 for a "twice daily" application.

13. Use of a composition as defined in any one of claims 1 to 6 or 11, or use of a dietary supplement as defined in any one of claims 7 to 10 for
(a) supporting a consumer's well-being; or
(b) alleviating a consumer's hangover or hangover symptoms; or
(c) protecting a consumer's liver function; or
(d) maintaining a consumer's liver function; or
of a composition as defined in any one of claims 1 to 6 or 11, or use of a dietary supplement as defined in any one of claims 7 to 10 for
(a) supporting a consumer's well-being; or
(b) alleviating a consumer's hangover or hangover symptoms; or
(c) protecting a consumer's liver function; or
(d) maintaining a consumer's liver function;
and wherein the consumer is a human; or
of a composition as defined in any one of claims 1 to 6 or 11, or use of a dietary supplement as defined in any one of claims 7 to 10 for
(a) supporting a consumer's well-being; or
(b) alleviating a consumer's hangover or hangover symptoms; or
(c) protecting a consumer's liver function; or
(d) maintaining a consumer's liver function;
wherein the consumer is a human, and wherein the liver has been or is or will be subjected to alcohol.

14. Composition as defined in any one of claims 1 to 6 or 11 for use in the
(a) prevention of detrimental effects in a consumer's liver caused by alcohol; or
(b) treatment of detrimental effects in a consumer's liver caused by alcohol.

15. Pharmaceutical composition, comprising the composition as defined in claim 14.

## Patentansprüche

1. Zusammensetzung, die mindestens die Komponenten (i) bis (iii) umfasst:
(i) Cholin in einer Menge von 70 - 99 Gew.-%,
(ii) ein oder mehrere Vitamine in einer Menge von 0,5 bis 25 Gew.-%,
(ii) Zink in einer Menge von 0,1 bis 10 Gew.-%,
wobei die Gesamtmenge an Cholin, des einen oder der mehreren Vitamine und des Zinks 100 Gew.-% beträgt.

2. Zusammensetzung nach Anspruch 1, enthaltend mindestens die Komponenten (i) bis (iii):
(i) Cholin in einer Menge von 80 - 99 Gew.-%,
(ii) ein oder mehrere Vitamine in einer Menge von 0,5 bis 20 Gew.-%,
(iii) Zink in einer Menge von 0,1 - 3 Gew.-%; oder
umfassend mindestens die Komponenten (i) bis (iii):
(i) Cholin in einer Menge von 85 - 99 Gew.-%,
(ii) ein oder mehrere Vitamine in einer Menge von 0,5 bis 12 Gew.-%,
(iii) Zink in einer Menge von 0,1 - 3 Gew.-%.

3. Zusammensetzung nach einem der vorhergehenden Ansprüche, wobei das eine oder die mehreren Vitamine ausgewählt sind aus der Gruppe bestehend aus Vitamin C, Vitamin E, Vitamin B6, Vitamin B9 oder einer Kombination von zwei oder mehr davon.

4. Zusammensetzung nach Anspruch 3, wobei das eine oder die mehreren Vitamine ausgewählt sind aus der Gruppe bestehend aus Vitamin C, Vitamin E, Vitamin B6, Vitamin B9 oder einer Kombination von zwei oder mehr davon, und wobei das eine oder die mehreren Vitamine eine Kombination von Vitamin C, Vitamin E, Vitamin B6 und Vitamin B9 sind; oder
wobei das eine oder die mehreren Vitamine ausgewählt sind aus der Gruppe bestehend aus Vitamin C, Vitamin E, Vitamin B6, Vitamin B9 oder einer Kombination von zwei oder mehr davon, und wobei das eine oder die mehreren Vitamine eine Kombination von Vitamin C, Vitamin E, Vitamin B6 und Vitamin B9 sind, und wobei
(i) die Cholinmenge im Bereich von 85 - 95 Gew.-% liegt,
(ii) die Kombination von Vitamin C, Vitamin E, Vitamin B6 und Vitamin B9 4 - 12 Gew.-% beträgt,
(iii) die Zinkmenge im Bereich von 0,2 - 3 Gew.-% liegt; oder
wobei
(i) die Cholinmenge im Bereich von 87 - 95 Gew.-% liegt,
(ii) die Menge an
Vitamin C 4 - 8 Gew.-% beträgt,
Vitamin E 0,5 - 2 Gew.-% beträgt,
Vitamin B6 0,05 - 0,4 Gew.-% beträgt.
Vitamin B9 0,01 - 0,1 Gew.-% beträgt.

5. Zusammensetzung nach Anspruch 3, wobei das eine oder die mehreren Vitamine eine Kombination aus Vitamin E, Vitamin B6 und Vitamin B9 sind;
oder
wobei das eine oder die mehreren Vitamine eine Kombination aus Vitamin E, Vitamin B6 und Vitamin B9 sind, und wobei
(i) die Cholinmenge im Bereich von 90 - 99 Gew.-% liegt,
(ii) die Kombination von Vitamin E, Vitamin B6 und Vitamin B9 0,5 - 8 Gew.-% beträgt,
(iii) die Zinkmenge im Bereich von 0,2 - 2 Gew.-% liegt; oder
wobei
(i) die Cholinmenge im Bereich von 92 - 99 Gew.-% liegt,
(ii) die Menge an
Vitamin E 0,5 - 6 Gew.-% beträgt,
Vitamin B6 0,05 - 0,4 Gew.-% beträgt,
Vitamin B9 0,01 - 0,1 Gew.-% beträgt,
(iii) die Zinkmenge im Bereich von 0,2 - 2 Gew.-% liegt.

6. Zusammensetzung nach einem der vorhergehenden Ansprüche, wobei mindestens 50 Gew.-% oder mindestens 60 Gew.-% oder mindestens 70 Gew.-% oder mindestens 80 Gew.-% oder mindestens 90 Gew.-% oder 100 Gew.-% der Zusammensetzung aus den Komponenten (i) bis (iii) bestehen, bezogen auf das Gesamtgewicht der Zusammensetzung, wenn die Zusammensetzung in fester Form vorliegt; oder
wobei 1 bis 5 Gew.-% oder 5 bis 10 Gew.-% oder 10 bis 15 Gew.-% oder 15 bis 20 Gew.-% oder 20 bis 25 Gew.-% oder 25 bis 30 Gew.-% oder 30 bis 35 Gew.-% oder 35 bis 40 Gew.-% oder 40 bis 45 Gew.-% oder 45 bis 50 Gew.-% oder 50 bis 70 Gew.-% der Zusammensetzung aus den Komponenten (i) bis (iii) beseht, bezogen auf das Gesamtgewicht der Zusammensetzung, wenn die Zusammensetzung in flüssiger Form vorliegt.

7. Nahrungsergänzungsmittel, umfassend die Zusammensetzung nach einem der vorhergehenden Ansprüche.

8. Nahrungsergänzungsmittel nach Anspruch 7 in Form eines Pulvers oder
in Form eines Pulvers, wobei das Pulver in einer Stick-Packung vorgesehen ist.

9. Nahrungsergänzungsmittel nach Anspruch 8 in Form eines Pulvers, wobei das Pulver in einer Stick-Packung vorgesehen ist, und wobei die Stick-Packung mindestens zwei Kammern aufweist, wobei das Vitamin C und Cholin in verschiedenen Kammern vorliegen, wenn die Zusammensetzung Vitamin C umfasst.

10. Nahrungsergänzungsmittel nach Anspruch 7 in Form einer Tablette, einer Kapsel, einer Brausetablette oder einer Lösung oder Emulsion.

11. Zusammensetzung nach einem der Ansprüche 1 bis 6 oder Nahrungsergänzungsmittel nach einem der Ansprüche 7 bis 10, wobei die Gegenwart von
(A) Lactose oder Zutaten auf der Basis von Lactose oder
(B) Zutaten, die nicht unter den arabischen Begriff "halal" fallen, oder
(C) Zutaten, die nicht unter den jüdischen Begriff "koscher" fallen
ausgeschlossen ist.

12. Verwendung eines Nahrungsergänzungsmittels gemäß Anspruch 9 für eine "einmal täglich"-Anwendung oder Verwendung eines Nahrungsergänzungsmittels gemäß Anspruch 10 für eine "zweimal täglich"-Anwendung.

13. Verwendung einer Zusammensetzung nach einem der Ansprüche 1 bis 6 oder 11 oder Verwendung eines Nahrungsergänzungsmittels nach einem der Ansprüche 7 bis 10 für
(a) die Unterstützung des Wohlbefindens eines Konsumenten; oder
(b) die Linderung der Kater- oder Kater-Symptome eines Konsumenten; oder
(c) den Schutz der Leberfunktion eines Konsumenten; oder
(d) die Aufrechterhaltung der Leberfunktion eines Konsumenten; oder
einer Zusammensetzung wie in einem der Ansprüche 1 bis 6 oder 11 definiert, oder Verwendung eines Nahrungsergänzungsmittels wie in einem der Ansprüche 7 bis 10 definiert, für
(a) die Unterstützung des Wohlbefindens eines Konsumenten; oder
(b) die Linderung der Kater- oder Kater-Symptome eines Konsumenten; oder
(c) den Schutz der Leberfunktion eines Konsumenten; oder
(d) die Aufrechterhaltung der Leberfunktion eines Konsumenten;
und wobei der Konsument ein Mensch ist; oder
einer Zusammensetzung wie in einem der Ansprüche 1 bis 6 oder 11 definiert, oder Verwendung eines Nahrungsergänzungsmittels wie in einem der Ansprüche 7 bis 10 definiert, für
(a) die Unterstützung des Wohlbefindens eines Konsumenten; oder
(b) die Linderung der Kater- oder Kater-Symptome eines Konsumenten; oder
(c) den Schutz der Leberfunktion eines Konsumenten; oder
(d) die Aufrechterhaltung der Leberfunktion eines Konsumenten;
wobei der Konsument ein Mensch ist und die Leber Alkohol unterworfen ist oder unterworfen sein wird.

14. Zusammensetzung wie in einem der Ansprüche 1 bis 6 oder 11 definiert zur Verwendung bei der
(a) Vermeidung von nachteiligen Wirkungen in der Leber eines Konsumenten verursacht durch Alkohol; oder
(b) Behandlung von nachteiligen Wirkungen in der Leber eines Konsumenten verursacht durch Alkohol.

15. Pharmazeutische Zusammensetzung, umfassend die Zusammensetzung nach Anspruch 14.

## Revendications

1. Composition comprenant au moins les composants (i) à (iii):
(i) de la choline en une quantité de 70 à 99 % en poids,
(ii) une ou plusieurs vitamines en une quantité de 0,5 à 25 % en poids,
(iii) du zinc en une quantité de 0,1 à 10 % en poids,
dans laquelle la quantité totale de choline, de la ou des vitamines et du zinc représente 100 % en poids.

2. Composition selon la revendication 1, comprenant au moins les composants (i) à (iii) :
(i) de la choline en une quantité de 80 à 99 % en poids,
(ii) une ou plusieurs vitamines en une quantité de 0,5 à 20 % en poids,
(iii) du zinc en une quantité de 0,1 à 3 % en poids ; ou
comprenant au moins les composants (i) à (iii) :
(i) de la choline en une quantité de 85 à 99 % en poids,
(ii) une ou plusieurs vitamines en une quantité de 0,5 à 12 % en poids,
(iii) du zinc en une quantité de 0,1 à 3 % en poids.

3. Composition selon l'une quelconque des revendications précédentes, dans laquelle la ou les vitamines sont choisies dans l'ensemble constitué par la vitamine C, la vitamine E, la vitamine B6, la vitamine B9, ou une combinaison de deux ou plus de celles-ci.

4. Composition selon la revendication 3, dans laquelle la ou les vitamines sont choisies dans l'ensemble constitué par la vitamine C, la vitamine E, la vitamine B6, la vitamine B9, ou une combinaison de deux ou plus de celles-ci, et dans laquelle la ou les vitamines sont une combinaison de vitamine C, de vitamine E, de vitamine B6, et de vitamine B9 ; ou
dans laquelle la ou les vitamines sont choisies dans l'ensemble constitué par la vitamine C, la vitamine E, la vitamine B6, la vitamine B9, ou une combinaison de deux ou plus de celles-ci, et dans laquelle la ou les vitamines sont une combinaison de vitamine C, de vitamine E, de vitamine B6, et de vitamine B9, et dans laquelle
(i) la choline représente 85 à 95 % en poids,
(ii) la combinaison de vitamine C, de vitamine E, de vitamine B6, et de vitamine B9 représente 4 à 12 % en poids,
(iii) le zinc représente 0,2 à 3 % en poids ; ou
dans laquelle
(i) la choline représente 87 à 95 % en poids,
(ii) la vitamine C représente 4 à 8 % en poids,
la vitamine E représente 0,5 à 2 % en poids,
la vitamine B6 représente 0,05 à 0,4 % en poids,
la vitamine B9 représente 0,01 à 0,1 % en poids.

5. Composition selon la revendication 3, dans laquelle la ou les vitamines sont une combinaison de vitamine E, de vitamine B6, et de vitamine B9 ; ou
dans laquelle la ou les vitamines sont une combinaison de vitamine E, de vitamine B6, et de vitamine B9, et dans laquelle
(i) la choline représente 90 à 99 % en poids,
(ii) la combinaison de vitamine E, de vitamine B6, et de vitamine B9 représente 0,5 à 8 % en poids,
(iii) le zinc représente 0,2 à 2 % en poids ; ou
dans laquelle
(i) la choline représente 92 à 99 % en poids,
(ii) la vitamine E représente 0,5 à 6 % en poids,
la vitamine B6 représente 0,05 à 0,4 % en poids,
la vitamine B9 représente 0,01 à 0,1 % en poids,
(iii) le zinc représente 0,2 à 2 % en poids.

6. Composition selon l'une quelconque des revendications précédentes, dans laquelle au moins 50 % en poids ou au moins 60 % en poids ou au moins 70 % en poids ou au moins 80 % en poids ou au moins 90 % en poids ou 100 % en poids de la composition sont constitués des composants (i) à (iii), par rapport au poids total de la composition, lorsque la composition est sous une forme solide ; ou
dans laquelle 1 à 5 % en poids, ou 5 à 10 % en poids, ou 10 à 15 % en poids, ou 15 à 20 % en poids, ou 20 à 25 % en poids, ou 25 à 30 % en poids, ou 30 à 35 % en poids, ou 35 à 40 % en poids, ou 40 à 45 % en poids, ou 45 à 50 % en poids, ou 50 à 70 % en poids de la composition sont constitués des composants (i) à (iii), par rapport au poids total de la composition, lorsque la composition est sous une forme liquide.

7. Complément alimentaire comprenant la composition telle que définie dans l'une quelconque des revendications précédentes.

8. complément alimentaire selon la revendication 7 sous la forme d'une poudre, ou
sous la forme d'une poudre, laquelle poudre est contenue dans un bâton.

9. Complément alimentaire selon la revendication 8, sous la forme d'une poudre, dans lequel la poudre est contenue dans un bâton, et dans lequel le bâton a au moins deux compartiments, et dans lequel ladite vitamine C et la choline sont dans des compartiments différents lorsque la composition comprend de la vitamine C.

10. Complément alimentaire selon la revendication 7 sous la forme d'un comprimé, d'une capsule, d'un comprimé effervescent, ou d'une solution ou émulsion.

11. Composition selon l'une quelconque des revendications 1 à 6, ou complément alimentaire selon l'une quelconque des revendications 7 à 10, dans lequel la présence
(a) de lactose ou d'ingrédients à base de lactose, ou
(b) d'ingrédients qui ne rentrent pas dans le cadre du terme arabe « halal », ou
(c) d'ingrédients qui ne rentrent pas dans le cadre du terme juif « casher » est exclue.

12. Utilisation d'un complément alimentaire selon la revendication 9 pour une application « une fois par jour », ou utilisation d'un complément alimentaire selon la revendication 10 pour une application « deux fois par jour ».

13. Utilisation d'une composition selon l'une quelconque des revendications 1 à 6 et 11, ou utilisation d'un complément alimentaire selon l'une quelconque des revendications 7 à 10 pour
(a) améliorer le bien-être d'un consommateur ; ou
(b) soulager la xylostomiase d'un consommateur ou les symptômes de la xylostomiase ; ou
(c) protéger la fonction hépatique d'un consommateur ; ou
(d) maintenir la fonction hépatique d'un consommateur ; ou
utilisation d'une composition selon l'une quelconque des revendications 1 à 6 et 11, ou utilisation d'un complément alimentaire selon l'une quelconque des revendications 7 à 10 pour
(a) améliorer le bien-être d'un consommateur ; ou
(b) soulager la xylostomiase d'un consommateur ou les symptômes de la xylostomiase ; ou
(c) protéger la fonction hépatique d'un consommateur ; ou
(d) maintenir la fonction hépatique d'un consommateur ;
et dans laquelle le consommateur est un humain ; ou
utilisation d'une composition selon l'une quelconque des revendications 1 à 6 et 11, ou utilisation d'un complément alimentaire selon l'une quelconque des revendications 7 à 10 pour
(a) améliorer le bien-être d'un consommateur ; ou
(b) soulager la xylostomiase d'un consommateur ou les symptômes de la xylostomiase ; ou
(c) protéger la fonction hépatique d'un consommateur ; ou
(d) maintenir la fonction hépatique d'un consommateur ;
dans laquelle le consommateur est un humain, et dans laquelle le foie a été ou est ou sera soumis à de l'alcool.

14. Composition selon l'une quelconque des revendications 1 à 6 et 11 pour une utilisation dans
(a) la prévention d'effets néfastes causés par l'alcool sur le foie d'un consommateur ; ou
(b) le traitement d'effets néfastes causés par l'alcool sur le foie d'un consommateur.

15. Composition pharmaceutique comprenant la composition telle que définie dans la revendication 14.
